# EUROPEAN PATENT SPECIFICATION

(11) **EP 0 503 479 B1**
(45) Date of publication and mention of the grant of the patent: **26.06.1996**
(21) Application number: 92103775.0
(22) Date of filing: 05.03.1992
(51) Int. Cl.: A61L 15/16

(54) **Use of a fabric in manufacturing a cleansing, disinfectant and/or curative element**
Verwendung eines Gewebes in der Herstellung eines reinigenden, desinfizierenden oder heilenden Elements
Emploi d'un tissu dans la fabrication d'un élément d'assainissement, de désinfection ou de guérison

(30) Priority: 11.03.1991 IT BO910068
(43) Date of publication of application: 16.09.1992
(73) Proprietor: Occhi, Mara, I-44028 Poggio Renatico (Ferrara) (IT)
(72) Inventor: Fusi, Daniele, I-44100 Ferrara (IT)
(74) Representative: Modiano, Guido, Dr.-Ing.

(56) References cited:
- BE-A- 626 086
- WORLD PATENTS INDEX LATEST, Section PQ, Week 8340, Derwent Publications Ltd., London, GB; Class P32, AN 83-779716; & JP-A-58 143 749
- WORLD PATENTS INDEX LATEST, Section PQ, Week 8937, Derwent Publications Ltd., London, GB, Class P32, AN 89-266770; & JP-A-01 192 871
- ENGINEERING IN MEDICINE, vol. 7, no. 1, January 1978, LONDON, GB, pages 11-15; G.B. PARK ET AL.: "The design and evaluation of a burn wound covering"
- WORLD PATENTS INDEX LATEST, Section PQ, Week 9150, Derwent Publications Ltd., London, GB; Class P32, AN 91-365015; & JP-A-03 244 456

## Description

The present invention relates to a use of a fabric in manufacturing a cleansing, disinfectant and/or curative element shaped like a band or napkin for treating and preventing surface or deep organic alterations.

The severe problems caused by bedsores are known, and it is equally known that hygiene of the involved parts and everything that facilitates blood circulation contribute to avoid or improve the conditions of these sores for human as well as animal treatment.

It is also known that it is important, in case of immobilization of some body parts, to remove dirt and sweat from dirty and sweaty body parts in order to avoid the onset of skin sores or diseases.

In addition, it is known that keeping at a physiological temperature parts which are subjected to surface or deep organic alterations is decisive for the rapid healing of injuries, burns, hematomas, sprains and even fractures.

Known from BE-A-626 086 is the use of a polyacrylonitrile fiber fabric for the production of bandage like-elements for organic lesions like injuries and burns. The fabric is made of friezed yarns.

From JP-A-58 143 749 is known the use of a moulded article of polyacrylonitrile resin containing acidic radicals and antibiotics as a bandage or gauze.

The technical aim of the present invention is indeed to devise a new and original use of a fabric in manufacturing a bandage-like cleansing, disinfectant and curative element. This aim is achieved by the present use of a fabric made of polyacrylonitrile fibers and having a furry side and a smooth side, for manufacturing a cleansing, disinfectant and/or curative element shaped like a band or napkin for the treatment and prevention of surface or deep organic alterations by applying said element with his furry side on a diseased part.

Conveniently, said fibers are of the type commercially known by the name DUNOVA, a registered trademark of the company BAYER A.G..

Further peculiarities will become apparent from the detailed description of a method of preferred use of said fabric.

Said fabric is spun and carded and has the appearance of a sort of lamb-skin with a smooth side and a furry side.

It has been observed that the simple contact of this fabric with sore or variously injured regions keeps them sterile and dry at a substantially constant temperature: it has been observed that, perhaps due to electrostatic charges of the fabric, by placing the fabric on the diseased part of the body the patient's circulation in that part is stimulated, and this aids the healing of even deep organic alterations such as bone fractures, sprains or the like in a reduced time.

Experimentation performed so far entails the use of a napkin, made of said material, simply wet in water to remove dirt, sweat (salts), organic residuals (blood) or the like from the diseased part and then the application on the diseased part of a band or napkin made of said fabric placed with its furry side (so to speak) against the skin.

If it is necessary to increase absorbency characteristics in some regions, one may simply place two sheets on top of one another in said regions, without connecting them with sewings or the like, since even the mere presence of sewings can cause lines of sweat or dirt to remain in contact with the patient's skin.

The fabric used has extremely high absorbency, warmth and sterility characteristics.

It has been observed that the removal of organic residuals of any kind (blood, sweat, discharges, etc.) from the fabric is extremely easy, and that simple washing with neutral soap is capable of returning the fabric to optimum hygienic conditions; it has furthermore been observed that with washing, the absorbency and warmth characteristics tend to increase, since the washes remove from the fabric the film of substances applied to the fibers for the weaving steps.

Another characteristic which has become evident is the rapidity with which the fabric dries after distributing over its entire surface the wetness with which it has made contact: perhaps it is also for this reason that the fabric is extremely valid in curing and preventing bedsores, since it removes from the skin of the immobilized patient all the organic substances which produce laceration and thus the onset of sores on the skin.

Bandages and bands used on fractured parts have speeded up their healing, perhaps due to a combination of the thermal, electrostatic and sweat-removing effects which facilitates improved blood flow in the diseased part.

It has thus been observed that the invention achieves the intended aim.

The invention thus conceived is susceptible to numerous modifications and variations. Thus, for example, the manufacture of tubular closed bands of any size and dimensions, the manufacture of sheets which may also be of any size and dimensions and possibly the manufacture of gloves which can be used for cleaning the diseased part are provided for.

In practice, the shapes, dimensions and unit weight of the fabric may be any according to the requirements without thereby abandoning the scope of the protection of the following claims.

## Claims

1. Use of a fabric made of polyacrylonitrile fibers and having a furry side and a smooth side, for manufacturing a cleansing, disinfectant and/or curative element shaped like a band or napkin for the treatment and prevention of surface or deep organic alterations by applying said element with his furry side on a diseased part.

2. Use of a fabric according to claim 1, characterized in that said fabric is spun and carded.

## Patentansprüche

1. Verwendung eines Gewebes, das aus Polyacrylnitril-Fasern gebildet ist und eine pelzartige Seite und eine glatte Seite aufweist, zum Herstellen eines reinigenden, desinfizierenden und/oder heilenden Elements, welches wie ein Band oder eine Serviette geformt ist, für die Behandlung und Prävention einer Oberfläche oder von tiefen organischen Änderungen, indem das Element mit seiner pelzartigen Seite auf einen erkrankten Teil aufgelegt wird.

2. Verwendung eines Gewebes gemäß Anspruch 1, dadurch gekennzeichnet, daß das Gewebe gesponnen und kardiert ist.

## Revendications

1. Utilisation d'un tissu constitué de fibres de polyacrylonitrile et présentant un côté qui a l'aspect d'une fourrure et un côté sans plis, pour la fabrication d'un élément d'assainissement, de désinfection et/ou de guérison façonné sous forme d'une bande ou d'une serviette, pour le traitement et la prévention de modifications organiques profondes ou en surface en appliquant ledit élément avec son côté qui a l'aspect d'une fourrure sur une partie malade.

2. Utilisation d'un tissu conforme à la revendication 1, caractérisée en ce que ledit tissu est filé et cardé.
